# EUROPEAN PATENT APPLICATION

(11) **EP 3 061 805 A1**
(43) Date of publication of application: **31.08.2016**
(21) Application number: 13895929.1
(22) Date of filing: 24.10.2013
(51) Int. Cl.: C12M 1/00

(54) **AUTOMATED CULTURE DEVICE**

(71) Applicant: Hitachi, Ltd., Chiyoda-ku Tokyo 100-8280 (JP)
(72) Inventor: NAKAMURA, Taku, Tokyo 100-8280 (JP); NOZAKI, Takayuki, Tokyo 100-8280 (JP); TERADA, Koichi, Tokyo 100-8280 (JP); KIYAMA, Masaharu, Tokyo 100-8280 (JP); ZHOU, Guangbin, Tokyo 100-8280 (JP); SUGAYA, Masakazu, Tokyo 100-8280 (JP); MATSUOKA, Shizu, Tokyo 100-8280 (JP)
(74) Representative: MERH-IP Matias Erny Reichl Hoffmann Patentanwälte PartG mbB
(86) International application number: PCT/JP2013/078857
(87) International publication number: WO 2015/059799

(57) **Abstract**

Provided is an automated culture device in which the exchange of a culture medium or the exchange of a gas in a culture vessel that is taken from the device is not needed. The device is equipped with multiple culture vessels (500 to 530), flow channels respectively connected to the multiple culture vessels, a vessel selection unit (300) for controlling a liquid or a gas that is to be sent to the culture vessels through the flow channels, and a control unit (400) for controlling the vessel selection unit, wherein the control unit (400) detects the disconnection of some of the flow channels which correspond to some of the culture vessels among the multiple culture vessels and alters the manner of the control of the sending of the liquid or the gas.

## Description

### Technical Field

The present invention relates to an automated culture device for culturing a cell or a tissue.

### Background Art

Regenerative medicine for recovering the function of an internal organ by using a biological sample such as a regenerated tissue produced from a cell as a raw material is expected as a curative treating method for diseases which have had no treatments. Regenerative tissues are produced based on SOP (Standard Operational Procedure) by a person engaged in manufacture and having a professional cell culturing technology at a CPC (Cell Processing Center) which provides a clean manufacturing environment. Therefore, the production of the regenerated tissues requires huge amounts of labor costs, time and operational cost. Since all the production steps are performed manually, there is limitation to the amount of a regenerated tissue to be produced. Therefore, production costs become high, thereby preventing the spread of regenerative medical treatments.

To break through this current situation, the introduction of an automated culture device which automates part or all of the culturing step is desired. By carrying out the culturing step by means of an automated culture device and not manually, labor saving and cost reduction can be realized, thereby making mass-production possible. In addition, since the operation of the automated culture device is constant, it is expected that this contributes to the constant quality of a regenerated tissue obtained after production.

As background art in this technical field, there is, for example, Patent Literature 1. This literature discloses that, in a culture device having a plurality of culture chambers, to solve a problem that it is troublesome to store and take out an object to be cultured into and from a culture chamber as compared with a culture device having only one culture chamber, a pin having a connector (female) is connected to the ground so that when a control unit detects that the pin is connected to the ground at the time of mating a connector (male) so as to detect that the pin is situated at the mating position, the control unit supplies a gas, power and a signal to a culture chamber and when the control unit detects that the pin is not at the mating position, the control unit stops the supply of a gas, power and a signal.

### Citation List

### Patent Literature

PTL 1: Japanese Patent Application Laid-Open No. 2006-149232

### Summary of Invention

### Technical Problem

For the quality inspection of a cultured cell, there is SOP in which some of culture vessels are selected at random and cells are taken out from the culture vessels to be inspected. Since quality inspection includes a time-consuming step, culture vessels are taken out, for example, one day before the end of the culturing period determined by SOP. In this case, the automated culture device continues to carry out medium or gas exchange for culture vessels left after the removal of the above culture vessels. However, medium or gas exchange should not be carried out in the routes of the removed culture vessels. This is because the medium or the gas may leak into the device or may remain in tubes connecting a medium supplier to the culture vessels as the destinations of the medium and the gas are not existent.

In Patent Literature 1 described above, although gas supply to the removed culture vessel is stopped, the gas remains in a flow channel. Although Patent Literature 1 is silent about medium exchange, when it is carried out with the same constitution, it is considered that the medium remains in the flow path. When medium exchange is carried out for another culture vessel while the medium remains in the flow channel, this amount of the medium in the flow channel flows into the culture vessel, in addition to the amount of a medium to be exchanged, whereby the medium overflows from the culture vessel, thereby making it impossible to continue culture.

It is an object of the present invention to provide an automated culture device which solves the above problem and does not perform medium exchange or gas exchange for the removed culture vessel.

### Solution to Problem

To attain the above object, the present invention provides an automated culture device which comprises a plurality of culture vessels, flow channels connected to the respective culture vessels, a vessel selection unit for controlling a liquid or gas to be supplied to the culture vessels through the flow channels, and a control unit for controlling the vessel selection unit, wherein the control unit detects the disconnection of flow channels corresponding to some of the culture vessels to change the control of liquid supply or gas supply.

### Advantageous Effects of Invention

According to the present invention, since medium exchange or gas exchange is not carried out for the flow channels of the removed culture mediums, the medium or the gas does not leak into the device or does not remain in the flow channels.

### Brief Description of Drawings

Fig. 1 is a diagram showing the constitution of an automated culture device according to a first example.
Fig. 2 is a diagram showing the constitution of a vessel selection unit in the first example.
Fig. 3 is a diagram showing the constitution of a culture supernatant vessel selection unit in the first example.
Fig. 4 is a diagram showing that one of the culture vessels of the automated culture device of the first example has been removed.
Fig. 5 is a flow chart showing a method of controlling medium exchange in the first example.
Fig. 6 is a diagram showing the constitution of an automated culture device according to a second example.
Fig. 7 is a flow chart showing a method of controlling medium exchange in the second example.
Fig. 8 is a diagram showing the constitution of an automated culture device according to a third example.
Fig. 9 is a diagram showing an example of a screen into which the number of a culture vessel to be removed is input in the third example.
Fig. 10 is a diagram showing another example of a screen into which the number of a culture vessel to be removed is input by means of a reader in the third example.

### Description of Embodiments

Hereinafter, preferred embodiments of the present invention will be described with reference to the accompanying drawings.

### Example 1

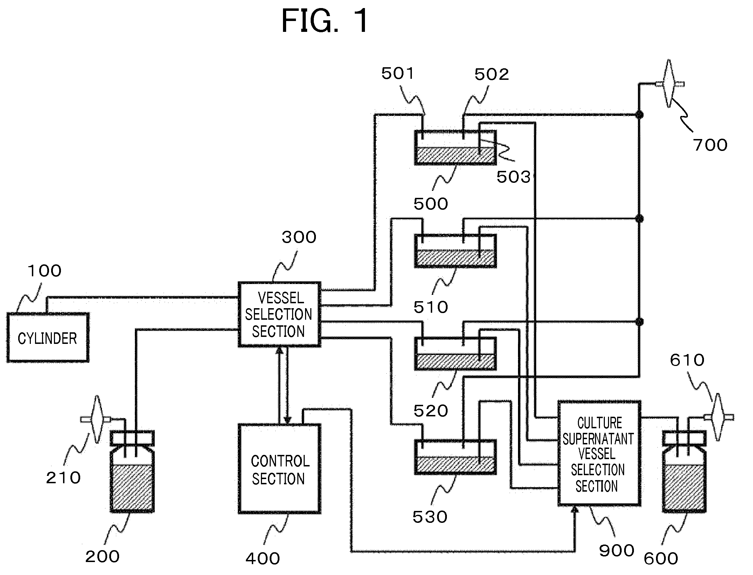

Fig. 1 is a schematic diagram showing an example of an automated culture device according to a first example. In Fig. 1, a cylinder 100 is filled with a gas required for the culture of a cell at a pressure higher than the atmospheric pressure and outputs the gas through a connected flow channel. The atmosphere of a medium used for culture generally contains 5% of CO2 so as to maintain a suitable pH value for culture. Therefore, the cylinder 100 is filled with a 5% CO2 gas which is an atmosphere containing 5% of CO2. A medium bottle 200 contains a medium to be exchanged and outputs it through a connected flow channel. An air filter 210 supplies the outside air to the medium bottle 200 therethrough in order to prevent the inside pressure of the medium bottle 200 from becoming a negative value when a medium is output at the time of medium exchange. Although the flow channels are not numbered in Fig. 1, it is needless to say that solid lines between the cylinder 100, the medium bottle 200, a vessel selection unit 300 for supplying a liquid or gas, culture vessels 500 to 530 and a culture supernatant vessel selection unit 900 indicate liquid and gas flow channels.

The vessel selection unit 300 for supplying a liquid or gas switches between the supply of a liquid and the supply of a gas based on the control of the control unit 400 and selects a culture vessel to be operated. Details of this constitution will be described hereinafter. The culture vessel 500 is sealed except for tubes 501, 502 and 503 connected thereto and stores a cell filled in a medium. The culture vessels 510, 520 and 530 have the same structure as the culture vessel 500. The culture supernatant collection selection unit 900 selects a culture vessel from which a culture supernatant is to be collected based on the control of the control unit 400. Details of this constitution will be described hereinafter. A culture supernatant bottle 600 stores a culture supernatant discharged from the culture vessels at the time of medium exchange.

An air filter 610 discharges the inside air to the outside therethrough in order to prevent the inside pressure of the culture supernatant bottle 600 from becoming a positive value when a culture supernatant flows into the culture supernatant bottle 600 at the time of medium exchange. An air filter 700 discharges a gas from a culture vessel to the outside at the time of gas exchange. A filter having a pore diameter of ,for example, 0.22 µm, is used as the air filters 210, 610 and 700 in order to prevent the contamination of the inside of a culture vessel by bacteria contained in the outside air. The flow channels of the medium and the gas of the automated culture device are closed by this constitution.

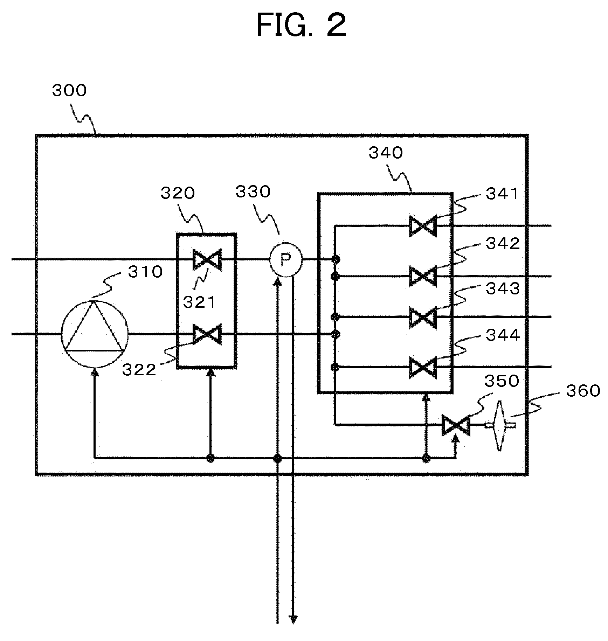

The constitution of the vessel selection unit 300 for supplying a liquid or gas in this example will be described with reference to Fig. 2. A pump 310 sucks out a medium from the medium bottle 200 when it receives an instruction from the control unit 400 and outputs it. An example of the pump 310 is a peristaltic pump which supplies a liquid by drawing a tube. A liquid/gas supply selection valve 320 is composed of a gas supply valve 321 and a liquid supply valve 322 both of which are generally closed. One of the gas supply valve 321 and the liquid supply valve 322 is opened based on an instruction from the control unit 400. A pressure sensor 330 measures the inside pressure of the tube and outputs the pressure value to the control unit 400. A vessel selection valve 340 is composed of valves 341, 342, 343 and 344 all of which are generally closed, and one of the valves is opened based on an instruction from the control unit 400. An exhaust valve 350 is opened or closed based on an instruction from the control unit 400. An air filter 360 prevents the inside of a culture vessel from being contaminated by bacteria contained in the outside air.

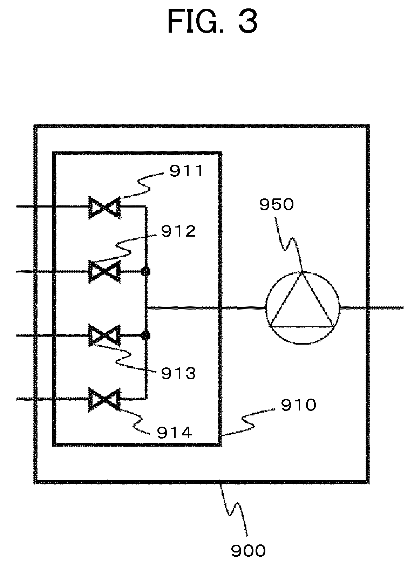

The constitution of the culture supernatant collection selection unit 900 of this example will be described with reference to Fig. 3. A vessel selection valve 910 is composed of valves 911, 912, 913 and 914 all of which are generally closed, and one of the valves is opened based on an instruction from the control unit 400. A pump 950 suctions out a culture supernatant from a culture vessel connected to the opened valve of the vessel selection valve 910 when it receives an instruction from the control unit 400 and outputs it to the culture supernatant bottle 600. An example of the pump 950 is the above-described peristaltic pump which supplies a liquid by drawing a tube.

The control unit 400 carries out medium exchange or gas exchange based on a culture schedule input by a person engaged in manufacture according to SOP at the start of cell culture. The culture schedule includes the times of starting medium exchange and gas exchange from the start to the end of cell culture. This control unit 400 can be constituted by using an ordinary computer which includes a controller, a central processing unit (CPU) for running programs, a memory for storing programs and data, and input and output units such as a display and a mouse.

The gas exchange operation of the automated culture device of this example will be described with reference to Fig. 1 and Fig. 2, taking the culture vessel 500 as an example. The control unit 400 first instructs the vessel section valve 340 to open the valve 341. Then, when the control unit 400 instructs the liquid/gas supply selection valve 320 to open the gas supply valve 321, the 5% CO2 gas contained in the cylinder 100 flows into the culture vessel 500 through the gas supply valve 321, the pressure sensor 330, the valve 341 and the tube 501 as the inside pressure of the cylinder 100 is higher than the atmospheric pressure. When the inside pressure of the culture vessel 500 becomes higher than the atmospheric pressure, the inside gas is extruded into the air filter 700 from the tube 502. After a predetermined time during which the amount of the gas determined by SOP is supplied, the control unit 400 instructs the liquid/gas supply selection valve 320 to close the gas supply valve 321 and the vessel selection valve 340 to close the valve 341.

A description is subsequently given of the medium exchange operation of the automated culture device of this example with reference to Fig. 1, Fig. 2 and Fig. 3, taking the culture vessel 500 as an example. Since a culture supernatant is stored in the culture vessel 500, the culture supernatant is transferred to the culture supernatant bottle 600. The control unit 400 instructs the vessel selection valve 910 to open the valve 911. Then, the control unit 400 instructs the operation of the pump 950. Since the valve 911 is opened, the culture supernatant of the culture vessel 500 is sucked out and moved into the culture supernatant bottle 600 through the tube 503. Along with the movement of the culture supernatant, the inside pressure of the culture vessel 500 drops, and the outside air flows into the culture vessel 500 from the air filter 700 through the tube 502. After a predetermined time during which the movement of the culture supernatant ends, the control selection 400 stops the operation of the pump 950 and instructs the vessel selection valve 910 to close the valve 911.

After the end of the movement of the culture supernatant in the culture vessel 500, the medium in the medium bottle 200 is supplied into the culture vessel 500. The control unit 400 instructs the vessel selection valve 340 to open the valve 341. Then, the control unit 400 instructs the liquid/gas supply selection valve 320 to open the liquid supply valve 322. Subsequently, the control unit 400 instructs the operation of the pump 310. The medium sucked out from the medium bottle 200 is supplied into the culture vessel 500 through the liquid supply valve 322, the valve 341 and the tube 501. After a predetermined time during which the amount of the medium determined by SOP is supplied, the control unit 400 stops the operation of the pump 310 and instructs the liquid/gas supply selection valve 320 to close the liquid supply valve 322 and the vessel selection valve 340 to close the valve 341.

According to SOP, the culture vessel is generally taken out one day before the end of the culturing period. As for the operation of taking out the culture vessel, two positions of each of the tubes connected to the culture vessel are heat-welded to be closed, and a part between the two positions is cut out to maintain the closing properties of a flow channel.

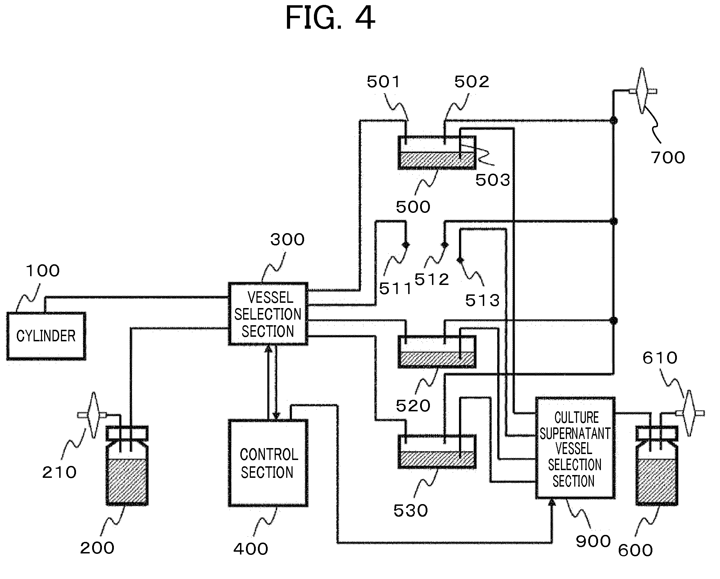

Fig. 4 shows the automated culture device of this example after the culture vessel 510 has been removed. The tubes 511, 512 and 513 are closed by heat-welding, thereby maintaining the closing properties of the flow channels.

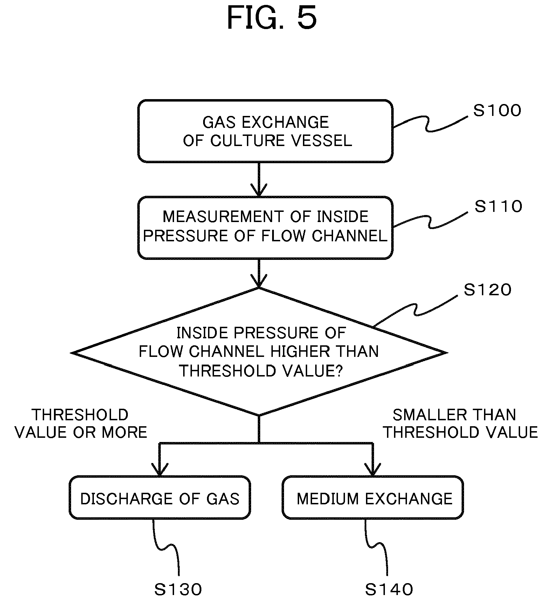

Operation from the time of starting the gas exchange of the culture vessel 510 specified in the culture schedule in the automated culture device in the state shown in Fig. 4 will be described with reference to the flow chart of Fig. 5. In Fig. 5, S100 means step 100. The control unit 400 carries out gas exchange for the culture vessel 510 (S100). Since the tube 511 is closed, the 5% CO2 gas of the cylinder 100 goes into the tube 511 and stops. The pressure from the cylinder 100 to the tube 511 rises to a value close to the inside pressure of the cylinder 100. The control unit 400 reads the value of the pressure sensor 330 to measure the inside pressure of the flow channel (S110).

When the inside pressure of the flow channel is lower than a threshold value close to the predetermined inside pressure of the cylinder 100, for example, 0.05 MPa, medium exchange is carried out in the same manner as medium exchange for the culture vessel 500 described previously at the time of starting medium exchange after the time of S100 specified in the culture schedule. When the inside pressure of the flow channel is not lower than the threshold value close to the predetermined inside pressure of the cylinder 100, the control unit 400 detects that the culture vessel 510 has been removed. When the control unit 400 detects that the culture vessel 510 has been removed, the gas in the flow channel is discharged (S130). The gas is discharged by opening the exhaust valve 350 after the gas supply valve 321 and the valve 342 are closed. The supply of the medium is not carried out without opening the liquid supply valve 322 at the time of medium exchange after the time of S100 specified in the culture schedule.

Thus, the control unit 400 of the automated culture device of this example has means of detecting the removed culture vessel so that it can perform the control of not carrying out medium exchange for the removed vessel. According to the constitution of this example, the medium does not remain in the flow channel, thereby eliminating the risk of overflowing the medium from the culture vessel.

As described above, in the constitution of the automated culture device of this example, the detection of the removed culture medium is carried out by the detection of the inside pressure of the flow channel by means of the pressure sensor 330, and the control unit 400 judges whether medium exchange is necessary or not. Since only a required amount of the medium is used in the constitution of this example, the volume of the medium stored in the medium bottle can be reduced, thereby making it possible to reduce the size of the medium bottle and also the size of the device. Further, since the amount of the expensive medium in use can be reduced, running cost for each time of cell culture can be cut.

In the automated culture device of this example, a cell in the culture vessel is observed through a microscope not shown in Fig. 1 and Fig. 4 during the culture period to check whether cell culture proceeds properly from an image from the microscope. The observation of the cell in the removed culture vessel can be eliminated by detecting the removed culture vessel by means of the above-described control unit 400. As a result, the death of a cell is not erroneously detected at the time of observing the cell through the microscope, whereby the processing system for detecting a cell is simplified and the detection accuracy of cell death is improved. Further, an image of the removed culture vessel is not stored in the memory, thereby eliminating the user's labor and time of sorting a cell image after the end of culture.

Although culture exchange was not carried out without opening the liquid supply valve 322 in Example 1 described above, the control unit 400 may control such that medium supply is not carried out without operating the pump 310 at the time of starting medium exchange specified in the culture schedule when it detects the removed culture vessel. When the medium is not supplied without opening the liquid supply valve 322, the medium slightly moves in the flow channel from the medium bottle 200 to the liquid supply valve 322. Although the medium moved into this flow channel may flow into an unexpected place at the time of the medium exchange of another culture vessel, since the medium is not supplied from the medium bottle 200 without operating the pump 310, the amount of the medium remaining in the flow channel can be made nil.

### Example 2

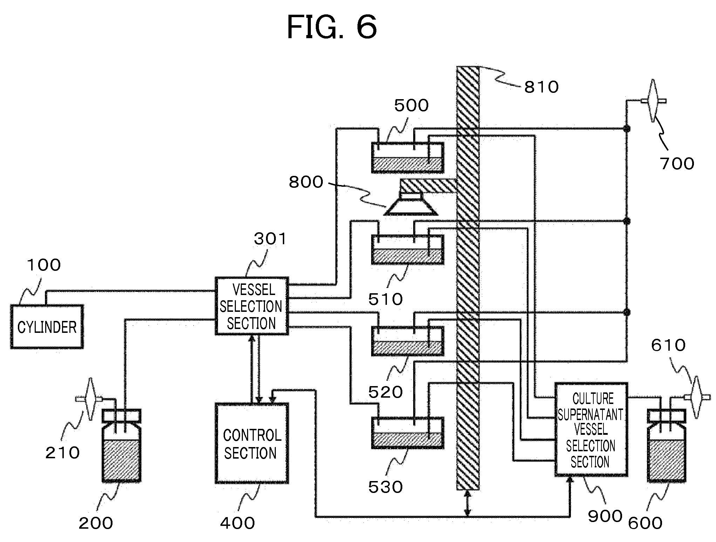

As a second example, a description is subsequently given of an automated culture device which detects the removal of a culture vessel from a photographed image of a microscope. Fig. 6 shows an example of this constitution. In Fig. 6, elements having the same operations as the constituent elements of the automated culture device shown in Fig. 1 and the constituent elements of the liquid/gas supply selection unit 300 shown in Fig. 2 are given the same reference numerals and their descriptions are omitted.

The vessel selection unit 301 for supplying a liquid or gas of the automated culture device shown in Fig. 6 does not includes the pressure sensor 330 out of the constituent elements of the liquid/gas supply selection unit 300 shown in Fig. 2 and is constituted such that the output of the gas supply valve 321 is directly connected to the vessel selection valve 340. A microscope 800 having a camera function photographs a cell in a culture vessel and outputs the image to the control unit 400. As an example of the microscope 800, there is a phase microscope suitable for the observation of a transparent cell. A microscope slider 810 supports the microscope 800 in the automated culture device and moves the microscope 800 to the position of one of the culture vessels 500 to 530 based on an instruction from the control unit 400. The control unit 400 carries out the movement of the microscope 800 and the observation of a cell in addition to medium exchange or gas exchange according to the culture schedule which is input by a person engaged in manufacture based on SOP at the start of cell culture.

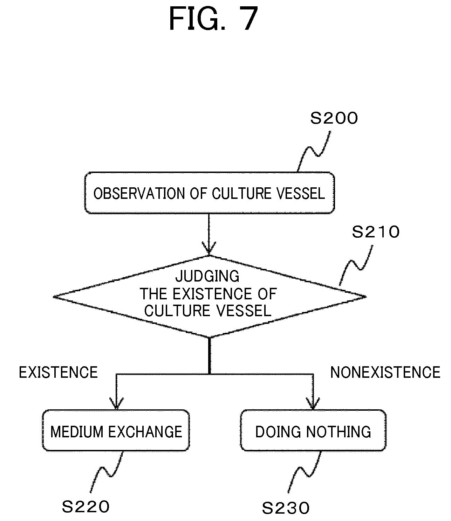

A method of judging whether a culture vessel is existent or not from a photographed image of the microscope by means of the automated culture device of this example will be explained with reference to the flow chart of Fig. 7. The control unit 400 instructs the microscope slider 810 to move the microscope 800 to the culture vessel 510 and the microscope 800 to photograph a cell in the culture vessel 510 after the end of movement (S200). The microscope 800 photographs a cell in the culture vessel 510 and transmits the photographed image to the control unit 400. The control unit 400 detects the shape of the cell from the photographed image and judges that the culture vessel exists when the cell is existent and that the culture vessel does not exist when the cell is not existent (S210).

As a result of the detection of the existence or nonexistence of the culture vessel, when the culture vessel exists, medium exchange is carried out at the time of starting medium exchange specified in the culture schedule. Medium exchange is carried out in the same manner as medium exchange for the culture vessel 500 described in Example 1. When the culture vessel does not exist, culture exchange is not carried out at the time of starting medium exchange specified in the culture schedule after the time of S200.

That is, the control unit 400 of this example has means of detecting the removed culture vessel and can control such that culture exchange is not carried out for the removed vessel. In this example, the medium does not remain in the flow channel, thereby eliminating the risk of overflowing the medium from the culture vessel like Example 1. Since only a required amount of the medium is used in this example, the volume of the medium stored in the medium bottle can be reduced, thereby making it possible to reduce the size of the medium bottle and also the size of the device. Further, since the amount of the expensive medium in use can be reduced, running cost for each time of cell culture can be cut.

Further, whether a culture vessel is existent or not can be judged from the result of a photographed image of the microscope, and gas exchange is not carried out for the removed vessel in this example. When gas exchange is carried out for the removed culture vessel, the gas remains in the flow channel. The remaining gas continues to apply excessive pressure to the end of the closed tube. If closing properties are lost by the broken tube, biochemical contamination may occur. Since a gas pressure change in the flow channel can be made small in this example, it is easy to maintain closing properties. It is also easy to design the strength of the tube and the pressure resistance performance of the tube at the time of disconnection.

When gas exchange is carried out for the route of the removed culture vessel, the inside pressure of the flow channel rises, whereby a big pressure change occurs at the time of the gas exchange of another culture vessel and becomes stress to a cell, thereby affecting the result of culture. Since a pressure change at the time of starting gas exchange is small in the constitution of this example, stress to a cell can be reduced and quality can be improved.

When gas exchange is carried out for the route of the removed culture vessel, the inside pressure of the flow channel rises, whereby it is erroneously detected that gas exchange fails for some reason. The processing of checking the gas pressure can be simplified and the detection accuracy of the gas pressure is enhanced in this example.

In the above explanation, an example in which a phase microscope is used as the microscope 800 of this example has been described. As described previously, the phase microscope is often mounted in an automated culture device to observe a transparent cell. The detection of a culture vessel is carried out by means of a microscope for cell observation, thereby making it possible to reduce the size of the device. Further, the cost of the device can be reduced.

Since a photographed image of the phase microscope is expressed only by a luminance value, it is difficult to detect color. The existence of a culture vessel may be detected by detecting the color of a medium by means of a stereomicroscope as the microscope 800 and checking whether the detected color falls within the range of changed color of phenolphthalein contained in an ordinary medium. To detect color, there are a method of detecting color from the RGB values of an image and a method of detecting color from HSV (Hue, Saturation, Value). Since color detection is easier than cell detection, the processing of detecting the existence of a culture vessel can be simplified and the detection accuracy can be improved. Further, the detection processing can be accelerated.

While the method of detecting a cell for the detection of a culture vessel has been described in this example, the end of a culture vessel may be detected by moving the microscope 800 having a camera function or a photographing unit such as a camera to the end of the culture vessel to carry out high-pass filter image processing on a photographed image for edge detection. This is because edge detection can be easily performed by high-pass filter processing when using an image of the end of a culture vessel whose flow channel has been disconnected at the end of the culture vessel connected to the flow channel such as a tube. Since high-pass filter processing has a small operation amount and is easier than cell detection, the detection of the existence of a culture vessel can be simplified and the detection accuracy can be improved. Further, the detection processing can be accelerated.

As means of detecting the existence of a culture vessel, a reader unit such as a bar code reader for reading a matrix type two-dimensional barcode code typified by a QR code (registered trademark) or a marker pattern such as bar code may be used. Which culture vessel has been removed can be detected and the detection accuracy can be improved by adding reference codes for distinguishing culture vessels 510 to 530 to the marker pattern. Further, the detection processing can be accelerated.

### Example 3

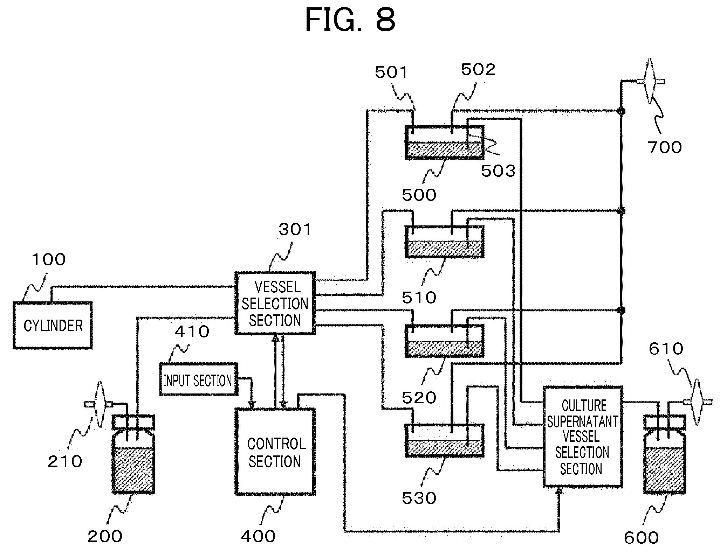

As a third example, an automated culture device in which a person engaged in manufacture who has removed a culture vessel inputs information on the removed culture vessel into the control unit will be described. Fig. 8 shows an example of this constitution. In Fig. 8, elements having the same operations as the constituent elements of the automated culture device shown in Fig. 4 and the constituent elements of the liquid/gas supply selection unit 300 shown in Fig. 2 are given the same reference numerals and their descriptions are omitted.

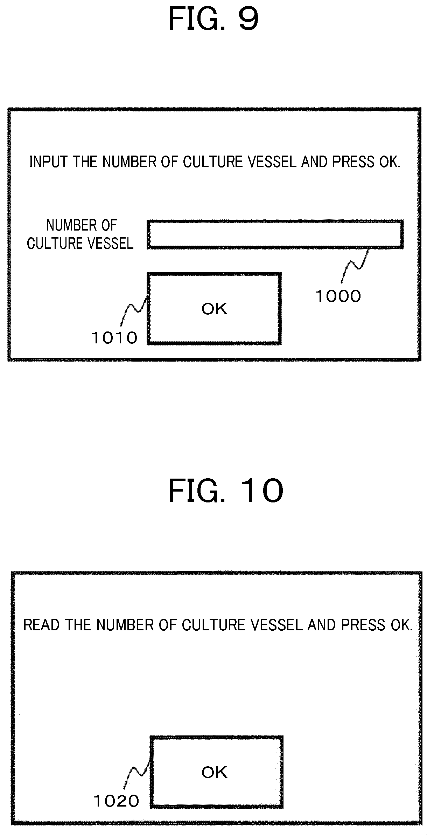

The input unit 410 of the automated culture device of Fig. 8 is used to input the number of one of the culture vessels 510 to 530 removed by a person engaged in manufacture into the control unit 400 and outputs the input information to the control unit 400. An input/output unit such as the display, keyboard and mouse of the computer described previously is used as the input unit 410. Fig. 9 shows an example of a screen for inputting the number of a culture vessel. The number of the removed culture vessel is input into the input part 1000 of the screen displayed on a display by using a keyboard and a mouse, and a button 1010 is pressed to complete input.

In the automated culture device of this example, the number of the removed culture vessel can be transmitted to the control unit 400 without being erroneously detected by the removal operation of a person engaged in manufacture and the explicit input of the number while he/she holds the removed culture vessel by hand, in addition to the effects shown in Example 1 and Example 2.

The number of the removed culture vessel may be input by causing the input unit 410 to read a marker pattern attached to the culture vessel. Fig. 10 shows an example of the screen for inputting the number of the culture vessel on a display which is a display unit. When a button 1020 is pressed after the number of the culture vessel is read by the input unit 410, input is completed. By using the marker pattern, the number of the culture vessel removed by a person engaged in manufacture is not erroneously input.

The number of the removed culture vessel may be input by attaching an electronic tag to the culture vessel to be read by the input unit 410. In this case, the input screen shown in Fig. 10 may be used as well. By using the electronic tag, a small culture vessel to which a marker pattern is difficult to be attached may be used. As compared with a case where a bar code is used, a large amount of information can be added.

Input into the input unit 410 may be made automatically and not manually by a person engaged in manufacture. For example, when an unshown contact sensor is installed on a floor for installing culture vessels and a culture vessel is removed by a person engaged in manufacture, the control unit 400 detects from the contact sensor that the culture vessel has been removed. In this case, the contact sensor functions as the input unit 410. According to this constitution, it is possible to prevent a person engaged in manufacture from forgetting the input of the number of the removed culture vessel.

Further, a detection sensor which detects the separation of a connector which is connected to a tube connected to a culture vessel when the culture vessel is removed may be installed to input the number of the culture vessel into the control unit 400. In this case, the detection sensor functions as the input unit 410. Since a person engaged in manufacture can remove the culture vessel while it is separated from the floor surface, working efficiency is improved.

Further, the input unit 410 may be installed in the opening section of an incubator storing the automated culture device so that an electronic tag is detected when a culture vessel leaves the incubator. Since the operation of removing the culture vessel is completed by taking out the culture vessel from the incubator, the timing of detecting the removed culture vessel can be made more accurate.

The detection of the removal of a culture vessel from the inside pressure of the flow channel shown in the first example may be combined with the input of the removed culture vessel shown in the third example. In Fig. 8, the control unit 400 judges whether medium exchange is necessary for a culture vessel represented by the culture vessel number input from the input unit 410 or not by using the method shown in Fig. 5. According to this constitution, when the number of the removed culture vessel is erroneously input, this can be notified to a user. The user can plan the next measure such as the resumption of the removal of the vessel or the continuation of the manual culture of the vessel removed erroneously. By checking only the number of the vessel input as the removed vessel, the check processing can be simplified and the amount of the gas in use can be reduced.

While preferred examples of the present invention have been described with reference to the accompanying drawings, it is needless to say that the present invention is not limited thereto. It is obvious that a person skilled in the art can arrive at variations and modifications within the scope of a technical idea described in claims, and it is to be understood that they fall within the technical scope of the present invention as a matter of course.

For example, the above examples have been described in detail to make it easy to understand the present invention and not limited to those having all the constitutions explained above. Part of the constitution of a certain example may be replaced by the constitution of another example, and the constitution of a certain example may be added to the constitution of another example.

Further, while an example in which a computer program is prepared to realize part or all of the above functions or the control unit has been described, it is needless to say that part or all of the functions or the control unit may be realized with hardware by designing with an integrated circuit.

### Reference Sings List

- 100:: cylinder
- 200:: medium bottle
- 210:: air filter
- 300:: vessel selection unit
- 310:: pump
- 320:: liquid/gas supply selection valve
- 330:: pressure sensor
- 340:: vessel selection valve
- 350:: exhaust valve
- 360:: air filter
- 400:: control unit
- 410:: input section
- 500:: culture vessel
- 600:: culture supernatant bottle
- 610:: air filter
- 700:: air filter
- 800:: microscope
- 810:: microscope slider
- 900:: culture supernatant collection selection section
- 910:: vessel selection valve
- 950:: pump

## Claims

1. An automated culture device comprising:
a plurality of culture vessels;
flow channels connected to the respective culture vessels;
a vessel selection unit for controlling a liquid or gas to be supplied into the culture vessels through the flow channels; and
a control unit for controlling the vessel selection unit,
wherein the control unit detects the disconnection of a flow channel corresponding to one of the culture vessels to change the control of the supply of the liquid or the supply of the gas.

2. The automated culture device according to claim 1 further comprising a pressure sensor for measuring the inside pressure of each flow channel,
wherein the control unit detects the disconnection of a flow channel based on a pressure value measured by the pressure sensor.

3. The automated culture device according to claim 1,
wherein the vessel selection unit includes a pump for supplying the liquid into the flow channels and the control unit controls the pump not to supply the liquid into a disconnected flow channel.

4. The automated culture device according to claim 1 further comprising a microscope for observing the culture vessels,
wherein the control unit detects the disconnection of a flow channel based on an image observed through the microscope.

5. The automated culture device according to claim 1 further comprising a marker pattern reading unit,
wherein the control unit detects the disconnection of a flow channel based on the output of the marker pattern reading unit.

6. The automated culture device according to claim 5,
wherein the marker pattern reading unit is a bar code reader.

7. The automated culture device according to claim 1 further comprising a photographing unit,
wherein the control unit detects the disconnection of a flow channel based on an image output from the photographing unit.

8. The automated culture device according to claim 7,
wherein the control unit detects the disconnection of a flow channel by high-pass filter processing an image of the end of a culture vessel output from the photographing unit.

9. The automated culture device according to claim 1 further comprising an input unit,
wherein the control unit detects the disconnection of a flow channel based on the number of a culture vessel input from the input unit.

10. The automated culture device according to claim 1 further comprising an input unit and a pressure sensor for measuring the inside pressure of a flow channel,
wherein the control unit detects the disconnection of the flow channel of a culture vessel corresponding to the number input from the input unit based on a pressure value measured by the pressure sensor.
